(19) Europäisches Patentamt
European Patent Office
Office européen des brevets

(11) Numéro de publication : **0 654 466 A1**

(12) **DEMANDE DE BREVET EUROPEEN**

(21) Numéro de dépôt : **94402611.1**

(22) Date de dépôt : **17.11.94**

(51) Int. Cl.[6] : **C07C 321/14,** C22B 3/00, C22B 60/02

(30) Priorité : **19.11.93 FR 9313856**

(43) Date de publication de la demande :
**24.05.95 Bulletin 95/21**

(84) Etats contractants désignés :
**BE DE ES GB IT**

(71) Demandeur : **COMPAGNIE GENERALE DES MATIERES NUCLEAIRES**
**2, rue Paul Dautier**
**F-78140 Velizy Villacoublay (FR)**

(72) Inventeur : **Guy, Alain**
**1, Rue Urgons**
**F-77135 Pontcarre (FR)**
Inventeur : **Lemaire, Marc**
**20, Rue Pierre Renaudel**
**F-50120 Equeurdreville (FR)**
Inventeur : **Foos, Jacques**
**33, Rue L. Scocard**
**F-91400 Orsay (FR)**
Inventeur : **Chomel, Rodolph**
**20, Rue du Buisson**
**F-84850 Camaret sur Aygues (FR)**
Inventeur : **Fache, Fabienne**
**118, Rue Alexis Perroncel**
**F-69100 Villeurbanne (FR)**
Inventeur : **Lellouche, Fabienne**
**163, Cours Tolstoi**
**F-69100 Villeurbanne (FR)**
Inventeur : **Estournel, Didier**
**Chemin de la Garaud N 1209**
**F-30200 Bagnols sur Ceze (FR)**

(74) Mandataire : **Dubois-Chabert, Guy**
**c/o BREVATOME**
**25, rue de Ponthieu**
**F-75008 Paris (FR)**

(54) **Nouveaux hémisépulcrands soufres et leur utilisation pour l'extraction de métaux, notamment du rhodium.**

(57) L'invention concerne de nouveaux hémisépulcrands soufrés et leur utilisation pour l'extraction de métaux, notamment du rhodium.

Ces hémisépulcrands soufrés répondent à la formule :

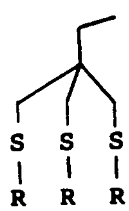

(I)

dans laquelle R représente un groupe choisi parmi

$-(CH_2)_pH$

$-(CH_2)_m-S-(CH_2)_pH$

$-(CH_2)_m-S-(CH_2)_n-S-(CH_2)_pH$

avec m et n qui peuvent être identiques ou différents représentant un nombre entier égal à 2,3,4 ou 5 et p étant un nombre entier allant de 1 à 18.

Ils peuvent être utilisés pour séparer le rhodium de solutions de retraitement de combustibles nucléaires irradiés par mise en contact de la solution aqueuse avec une phase organique immiscible contenant l'hémisépulcrand soufré afin d'extraire le rhodium dans la phase organique.

EP 0 654 466 A1

La présente invention a pour objet de nouveaux tripodands dénommés ci-après hémisépulcrands soufrés, utilisables en particulier pour séparer des métaux de solutions aqueuses.

De façon plus précise, elle concerne des hémisépulcrands soufrés capables de séparer les métaux tels que le rhodium, d'une solution aqueuse de retraitement de combustibles nucléaires irradiés.

Depuis plusieurs années, la technique la plus largement utilisée pour réaliser le retraitement des combustibles nucléaires irradiés consiste à dissoudre le combustible dans une solution nitrique, ce qui conduit à l'obtention d'une solution aqueuse nitrique contenant de l'uranium, du plutonium et des produits de fission. Après cette étape de dissolution, on met la solution aqueuse nitrique ainsi obtenue en contact avec un solvant organique pour extraire dans celui-ci l'uranium et le plutonium et les séparer de la majorité des produits de fission. On réextrait ensuite l'uranium et le plutonium dans une phase aqueuse, puis on sépare l'uranium du plutonium présent dans cette phase aqueuse au moyen d'un solvant organique.

Parmi les produits de fission restant dans la solution aqueuse de dissolution après l'extraction de l'uranium et du plutonium, on trouve des métaux précieux tels que le rhodium, le ruthénium et le palladium, en concentrations non négligeables, qu'il serait intéressant de récupérer à ce stade.

Dans de telles solutions, on peut extraire le palladium au moyen de ligands du type thioéthers, comme il est décrit dans le document FR-A- 2 662 159.

Dans le cas du ruthénium, on peut séparer celui-ci par volatilisation et adsorption sur de la polyvinylpyridine comme il est décrit dans FR-A- 2 688 335.

Dans le cas du rhodium, plusieurs solutions peuvent être envisagées pour extraire celui-ci des solutions aqueuses de retraitement car on sait que les extractants soufrés comme les thioéthers, les acides sulfoniques et les amines soufrées, ainsi que les amines tertiaires sont capables d'extraire le rhodium, comme il est décrit par Patel et al dans Solvent Extraction and Ion Exchange, 5(4), 1987, p. 633-647, et par Gorski et al dans Isotopenpraxis, 24, 1988, p. 200-206.

Cependant, aucune de ces méthodes ne donne des performances satisfaisantes pour la récupération du rhodium à partir des solutions de retraitement de combustibles nucléaires irradiés, notamment en raison de la cinétique trop lente d'extraction du rhodium qui interdit leur utilisation en milieu radioactif.

La présente invention a précisément pour objet de nouveaux hémisépulcrands qui permettent d'extraire le rhodium avec des rendements très élevés, à partir de solutions aqueuses issues du retraitement de combustibles irradiés telles que des concentrats de produits de fission.

Selon l'invention, l'hémisépulcrand soufré répond à la formule :

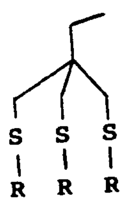

(I)

dans laquelle R représente un groupe choisi parmi

- $(CH_2)_pH$
- $(CH_2)_m$-S-$(CH_2)_pH$
- $(CH_2)_m$-S-$(CH_2)_n$-S-$(CH_2)_pH$

avec m et n qui peuvent être identiques ou différents représentant un nombre entier égal à 2,3,4 ou 5 et p étant un nombre entier allant de 1 à 18.

Les hémisépulcrands soufrés conformes à l'invention peuvent ainsi être du type tridenté, du type hexadenté ou du type nonadenté. Ce sont des ligands capables de complexer certains métaux, en particulier le rhodium et le ruthénium, et de permettre leur extraction sélective à partir de solutions aqueuses provenant du retraitement de combustibles nucléaires irradiés.

Ces hémisépulcrands peuvent être préparés par un procédé analogue à celui décrit par Thorne et al dans Inorg. Chem., 25, 1986, p. 3848-3850, et Cooper dans Pure & Appl. Chem., vol. 62, n° 6, 1990, p. 1123-1125, pour la préparation d'un hémisépulcrand hexadenté : le 1,1,1-tris[2-(méthylthio)éthylthio)]-méthyl]éthane qui présente des propriétés complexantes vis-à-vis de nombreux métaux tels que Ni, Co, Fe, Pd, Ru et Rh.

Aussi, l'invention a également pour objet un procédé de préparation d'un hémisépulcrand de formule (I) :

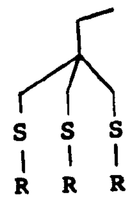

(I)

dans laquelle R représente $-(CH_2)_pH$ avec p étant un nombre entier allant de 1 à 18, caractérisé en ce que l'on fait réagir le tritosylate de 1,1,1-tris(hydroxyméthyl)propane avec un thiol de formule $H(CH_2)_p$-SH dans laquelle p a la signification donnée ci-dessus.

Dans le cas de la préparation d'hémisépulcrands hexadentés, c'est-à-dire d'hémisépulcrands de formule (I) dans laquelle R représente $-(CH_2)_m-S-(CH_2)_pH$ avec m=2,3,4 ou 5 et p étant un nombre entier de 1 à 18, le procédé consiste à faire réagir un dithiol de formule $HS-(CH_2)_m-SH$ avec un halogénure de formule $X(CH_2)_pH$ dans lesquelles m et p ont les significations données ci-dessus et X représente I ou Br, puis à faire réagir le produit obtenu avec le tritosylate de 1,1,1-tris(hydroxyméthyl)propane.

Dans le cas des hémisépulcrands nonadentés, c'est-à-dire des hémisépulcrands de formule I dans laquelle R représente $-(CH_2)_m-S-(CH_2)_n-S-(CH_2)_pH$ avec m, n et p ayant les significations données ci-dessus, on peut obtenir ces composés par réaction d'un dithiol de formule $HS-(CH_2)_m-S-(CH_2)_n-SH$ avec un halogénure de formule $X(CH_2)_pSH$ dans lesquelles m, n et p ont les significations données ci-dessus et X représente I ou Br, suivie de la réaction du produit obtenu avec le tritosylate de 1,1,1-tris(hydroxyméthyl)propane.

Le tritosylate de 1,1,1-tris(hydroxyméthyl)propane peut être préparé par un procédé classique tel que celui décrit par Ouchi et al dans Bull. Chem. Soc. Jpn.,63, (1990), p. 1260-1262.

Les hémisépulcrands soufrés de l'invention peuvent être utilisés pour la récupération de divers métaux, en particulier du rhodium, du palladium, du ruthénium, de l'uranium et/ou du plutonium à partir de solutions aqueuses telles que les solutions issues du retraitement des combustibles nucléaires irradiés.

En effet, ces hémisépulcrands sont de bons extractants du rhodium et ils présentent de plus une bonne tenue à la radiolyse et à l'hydrolyse nitrique, ce qui les rend particulièrement adaptés au traitement de solutions issues du retraitement des combustibles irradiés.

Aussi, l'invention a également pour objet un procédé de séparation d'au moins un métal choisi parmi Rh, Pd, Ru, U et Pu, notamment du rhodium, présents dans une solution aqueuse issue du retraitement des combustibles nucléaires irradiés, qui consiste à mettre en contact la solution aqueuse avec une phase organique immiscible comprenant un hémisépulcrand soufré conforme à l'invention, pour extraire le ou les métaux dans la phase organique, et séparer de la solution aqueuse la phase organique contenant le ou les métaux extraits.

Selon l'invention, la phase organique immiscible à l'eau peut être une phase liquide ou une phase solide. Dans le cas où cette phase organique est une phase liquide, celle-ci comprend généralement un diluant organique qui peut être choisi, par exemple, parmi les solvants chlorés tels que le chloroforme, les hydrocarbures tels que l'heptane, le tétrapropylène hydrogéné, les nitriles tels que le benzonitrile, l'hexanenitrile, l'octane 1,8-dicarbonitrile et les alcools tels que l'hexanol.

De préférence, on utilise le chloroforme, le benzonitrile, l'hexanenitrile ou l'hexanol.

La concentration en hémisépulcrand soufré de la phase organique peut varier dans une large gamme et elle dépend en particulier de l'hémisépulcrand et du diluant organique utilisés.

En effet, cette concentration doit être telle que l'on obtienne une phase organique homogène sans cristallisation de l'hémisépulcrand soufré ou des complexes hémisépulcrands soufrés-métaux extraits.

Généralement, on utilise une concentration en hémisépulcrand soufré allant de 0,025 à 0,5 mol/l.

Lorsqu'on utilise une phase organique liquide, on peut mettre en contact celle-ci avec la solution aqueuse et séparer ensuite les deux phases dans des appareillages classiques, par exemple dans des colonnes d'échange à co-courant ou à contre-courant telles que les colonnes pulsées, dans des mélangeurs-décanteurs ou encore dans des extracteurs centrifuges. On opère généralement à la pression et à la température ambiantes.

La durée de mise en contact dépend en particulier du métal à extraire, c'est-à-dire de sa cinétique d'extraction.

Ainsi, lorsqu'on veut extraire le palladium seul, on peut utiliser des durées très faibles, par exemple une durée de 60 minutes, car le palladium est extrait très vite dans la phase organique.

En revanche, pour extraire le rhodium, on utilise des durées beaucoup plus longues car sa cinétique d'extraction est beaucoup plus lente, par exemple une durée de 5 à 20h.

Les solutions aqueuses contenant les métaux précieux à extraire sont de préférence des solutions nitriques, ayant par exemple une concentration en acide nitrique de 0,5 à 4mol/l.

Après extraction des métaux dans la phase organique, ceux-ci peuvent être récupérés par lavage au moyen de $HNO_3$ et calcination.

Lorsqu'on extrait simultanément plusieurs métaux y compris du rhodium, on peut aussi soumettre la phase organique ayant extrait le ou les métaux à un lavage par une solution nitrique ayant une teneur en $HNO_3$ de 0,1 à 1mol/l pour réextraire certains métaux et purifier le rhodium qui reste en phase organique. Le rhodium peut être récupéré ensuite par calcination.

Lorsque l'on utilise une phase organique solide, celle-ci peut être constituée par des phases organiques ou minérales sur lesquelles sont greffés les hémisépulcrands par l'intermédiaire d'un atome de soufre.

Les solutions aqueuses issues du retraitement des combustibles nucléaires irradiés peuvent être par exemple des effluents aqueux obtenus en fin de retraitement ou des concentrats de produits de fission obtenus après la séparation de l'uranium et du plutonium de la solution de dissolution.

Ces concentrats contiennent pratiquement tous les produits de fission et sont constitués généralement par une solution nitrique.

Dans ces concentrats, on peut récupérer le rhodium avec d'autres éléments ou bien soumettre tout d'abord le concentrat à des traitements en vue de séparer tout d'abord le palladium et le ruthénium, sans extraire le rhodium.

D'autres caractéristiques et avantages de l'invention apparaîtront mieux à la lecture des exemples suivants donnés bien entendu à titre illustratif et non limitatif, en référence au dessin annexé sur lequel

- la figure 1 est un diagramme représentant le pourcentage d'extraction du rhodium en fonction du temps (en h) par une phase liquide comprenant l'hémisépulcrand n° 4 conforme à l'invention, dans le benzonitrile,
- la figure 2 est un diagramme représentant le pourcentage d'extraction du rhodium en fonction du temps (en h) pour le même hémisépulcrand que celui de la figure 1 dans le chloroforme,
- la figure 3 représente de façon schématique le mode opératoire utilisé pour l'extraction des métaux présents dans un concentrat de produits de fission, et
- la figure 4 est un diagramme illustrant les cinétiques d'extraction de divers éléments par l'hémisépulcrand n° 4 conforme à l'invention, dans le benzonitrile.

**Exemple 1.** : Préparation du 3,3'-bis(2-thiaheptane)-5-thiadécane (composé n° 1).

Ce composé répond à la formule I de l'invention avec R = $C_5H_{11}$.

a) Préparation du tritosylate de 1,1,1-tris(hydroxyméthyl)propane.

La synthèse de ce composé correspond au schéma réactionnel suivant :

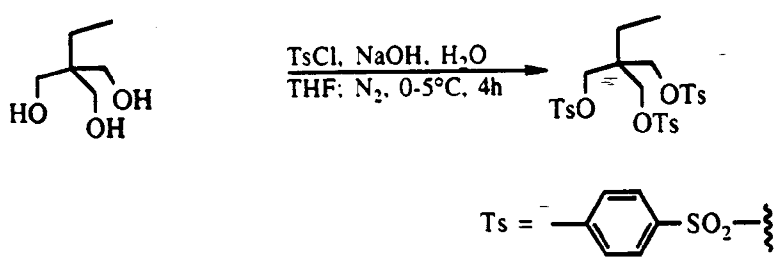


On dissout 500mmol de 1,1,1-tris(hydroxyméthyl)propane dans une solution comportant 300mmol de NaOH et 43ml d'eau. On rajoute à ce mélange refroidi à 0-5°C une solution de 180mmol de chlorure de tosyle dissous dans 43ml de tétrahydrofuranne (THF). Le mélange est agité pendant 4h en maintenant la température à 0-5°C sous atmosphère d'azote. Après décantation, on extrait la phase aqueuse au dichlorométhane et les phases organiques réunies sont lavées à l'acide chlorhydrique à 10% puis par de l'eau jusqu'à neutralité. Après séchage sur sulfate de magnésium, les phases organiques sont évaporées et le produit est obtenu avec un rendement de 62%.

b) Préparation du composé n° 1.

Cette préparation correspond au schéma réactionnel suivant :

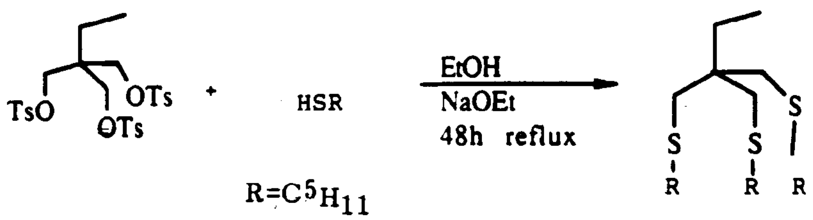


On dissout 59mmol d'hydroxyde de sodium, 8,4mmol du tritosylate préparé précédemment et 37mmol de pentanethiol dans 80ml d'éthanol et on porte au reflux pendant 48h sous argon. On évapore le solvant et on extrait le produit par un mélange de chlorure de méthylène et d'eau, puis on sèche sur $MgSO_4$. On purifie ensuite le produit par chromatographie sur colonne de silice (20 parts) en réalisant l'élution au moyen d'un mélange heptane-éther (10:1).

On obtient ainsi le composé n° 1, soit le 3,3'-bis(2-thiaheptyl)-5-thiadécane, avec un rendement de 86%.

Les caractéristiques du composé n° 1 sont les suivantes :

RMN $^1H$ (200MHz) : δ 0,83 (t, 3H, J = 7,5, H1), 0,92 (t, 9H, J = 7, H10), 1,37 (m, 12H, H8 et H9), 1,51 (q, 2H, J = 7,57, H2), 1,61 (m, 6H, H7), 2,55 (t, 6H, J = 7,3, H6), 2,66 (s, 6H, H4),.

$^{13}C$ (50MHz) : δ 7,83 Cl, 13,91 C10, 22,25 C9, 27,74 C2, 29,59 C8, 31,05 C7, 33,76 C6, 38,8 C4, 42,41 C3.

**Exemple 2** : Préparation du 3,3'-bis(2-thiadécyl)-5-thiatridécane (composé n° 2).

Ce composé répond à la formule I de l'invention avec R = $C_8H_{17}$.

Pour cette préparation, on suit le même mode opératoire que dans l'exemple 1, sauf que l'on utilise 37mmol d'octanethiol à la place du pentanethiol.

On obtient ainsi le composé n°2 avec un rendement de 75%.

Les caractéristiques du composé n° 2 sont les suivantes :

RMN $^1H$ (200MHz) : δ 0,85 (t, 3H, H1), 0,89 (t, 12H, H13), 1,33 (m, 36H, H2, H8, H12), 2,51 (t, 6H, J = 7,20, H6), 2,61 (s, 6H, H4).

$^{13}C$ (50MHz) : δ 7,95 Cl, 14,15 C13, 22,78 C12, 27,84 C2, 29,03, 29,23, 29,40, 30,11, 32 C7 à C11, 33,90 C6, 38,85 C4, 42,58 C3.

**Exemple 3** : Préparation du 3, 3'-bis(2,5-dithiatridécane)-5, 8-dithiahexadécane (composé n° 3)

Ce composé répond à la formule I de l'invention avec R représentant $-(CH_2)_2-S-(CH_2)_8H$.

On dissout 70mmol de 1, 2-éthanedithiol dans 150ml de diméthylformamide en présence de 80mmol de tert-butylate de potassium et 80mmol de bromooctane et on porte au reflux pendant 1h. On ajoute ensuite 14mmol du tritosylate préparé dans l'exemple 1a et 74mmol de t-butylate de potassium, et on porte le mélange au reflux pendant 48h. Après distillation des sous-produits de faible poids moléculaire, on purifie le composé n° 3 par chromatographie sur alumine neutre (20parts) en utilisant comme éluant le mélange heptane-éther (20:1).

On obtient ainsi le composé n° 3.

Les caractéristiques du composé n° 3 sont les suivantes :

RMN $^1H$ (200MHz) : δ 0,86 (m, 12H, H1, H16), 1,36 (m, 32H, H2, H11 à H15), 1,59 (m, 12H, H10), 2,54 (t, 6H, J = 7,28, H9), 2,69 (m, 18H, H4, H6, H7)

$^{13}C$ (50MHz) : δ 7,89 Cl, 14,10 C16, 22,64 C15, 27,71 C2, 28,89 à 29,73 C11 à C14, 31,80 à 33,69 C6, C7, C9, C10, 38,58 C4, 42,55 C3.

**Exemple 4** : Préparation du 3, 3'-bis(2,6-dithiatétradécane)-5,9-dithiaheptadécane (composé n° 4).

Ce composé répond à la formule I de l'invention avec R représentant $-(CH_2)_3-S-C_8H_{17}$.

Pour cette préparation, on suit le même mode opératoire que dans l'exemple 3, sauf que l'on utilise 70mmol de 1,3-propanedithiol au lieu de 70mmol de 1,2-éthanedithiol.

Les caractéristiques du composé n° 4 ainsi obtenu sont les suivantes :

RMN $^1H$ (200MHz) : δ 0,85 (t, 3H, J = 7,4, H1), 0,88 (t, 9H, J = 6,7, H17), 1,27 (m, 32H, H2, H12 à H16), 1,56 (m, 6H, H11), 1,86 (m, 6H, H7), 2,50 (t, 6H, J = 7,3, H10), 2,60 (t, 6H, J = 7,1, H8), 2,63 (s, 6H, H4), 2,64 (t, 6H, J = 7, H6)

$^{13}C$ (50MHz) : δ 7,93 Cl, 14,11 C17, 22,67 C16, 27,83 C2, 28,97, 29,23, 29,67, 29,72, 30,93 C11 à C15, 31,84,

32,21, 32,61 C6, C8, C10, 38,81 C4, 42,51 C3.

Exemple 5 : Préparation du 3,3'-bis (2,7-dithiapentadécane)-5,10-dithiaoctadécane (composé n° 5).

Ce composé répond à la formule I de l'invention avec R représentant $-(CH_2)_4-S-C_8H_{17}$.

Pour cette préparation, on suit le même mode opératoire que dans l'exemple 3, sauf que l'on utilise 70mmol de 1,4-butanedithiol au lieu de 70mmol de 1,2-éthanedithiol. On obtient ainsi le composé n° 4.

Les caractéristiques du composé n° 4 sont les suivantes :

RMN $^1H$ (200MHz) : δ 0,84 (t, 3H, J = 7,1, H1), 0,88 (t, 9H, J = 7,1, H17), 1,27 (m, 32H, H2, H13 à H16), 1,57 (m, 6H, H12), 1,69 (m, 12H, H7 H8), 2,50 (t, 6H, J = 7,3, H11) , 2,52 (t, 12H, J = 6,9, H6 H9) , 2,62 (s, 6H, H4).

$^{13}C$ (50MHz) : δ 7,93 Cl, 14,12 C18, 22,68 C17, 27,78 C2, 28,68 à 31,66 C7 C8 et C12 à C17, 31,86 32,15 et 33,32 C6 C9 C11, 38,73 C4, 42,48 C3.

Exemple 6 : Préparation du 3, 3'-bis (2,5,8-trithianonane)-5,8,11-trithiadodécane (composé n° 6).

Ce composé répond à la formule I de l'invention avec R représentant $-(CH_2)_2-S-(CH_2)_2-S-CH_3$.

Cette préparation correspond au schéma réactionnel suivant :

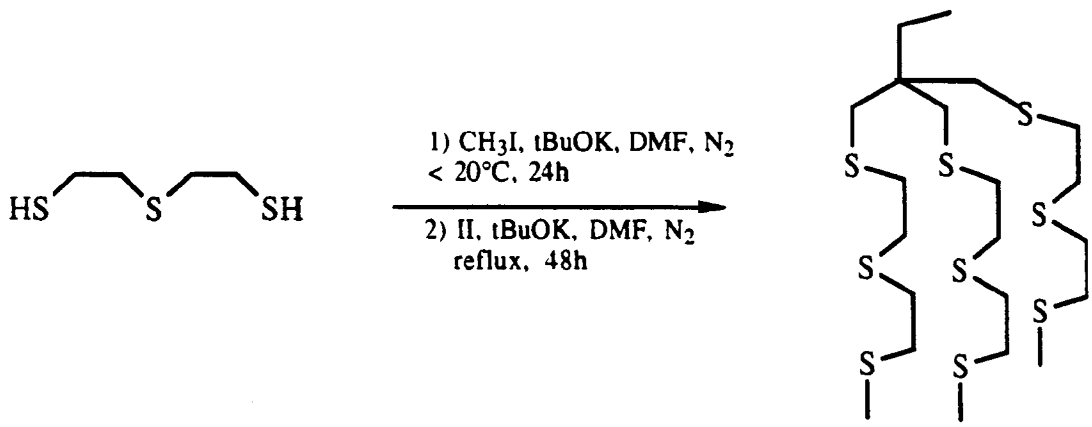


VIII

On ajoute 46mmol d'iodure de méthyle à 42mmol de 2,2'-thiodiéthanethiol et 46mmol de t-butylate de potassium dans 90mml de diméthylformamide (DMF), sous argon, à une température inférieure à 20°C. On maintient le milieu réactionnel dans ces conditions sous agitation pendant 24h, puis on lui ajoute 6mmol du tritosylate obtenu dans l'exemple la et 46mmol de t-butylate de potassium dans 40ml de DMF, sous argon, et on porte le mélange au reflux pendant 48h.

Après distillation des sous-produits de faible poids moléculaire, on purifie l'hémisépulcrand par chromatographie sur alumine neutre (20 parts) en utilisant comme éluant un mélange heptane-éther (20:1).

On obtient ainsi le composé n°6 avec un rendement de 17%.

Les caractéristiques du composé n° 6 sont les suivantes :

RMN $^1H$ (200MHz) : δ 0,86 (t, 3H, H1), 1,43 (q, 2H, H2), 2,16 (s, 9H, H12), 2,67 (s, 6H, H4), 2,78 (m, 24H, H6 H7 H9 H10).

$^{13}C$ (50MHz) : δ 7,88 Cl, 15,56 C12, 27,68 C2, 31,78 à 34,24 C6 C7 C9 C10, 38,57 C4, 42,54 C3.

Exemple 7 : Extraction du rhodium.

Dans cet exemple, on extrait le rhodium au moyen d'une phase organique liquide comprenant le composé n° 4 préparé dans l'exemple 4.

La solution aqueuse de rhodium est une solution d'acide nitrique 1,38N contenant 198,5mg/l de rhodium. La phase organique est une solution du composé n° 4 à une concentration de 0,0539 mol/l (4% en masse) dans le benzonitrile.

Pour réaliser la séparation, on met en contact 10ml de phase organique d'extraction avec lOml de la solution aqueuse de rhodium (III) en soumettant l'ensemble à une agitation magnétique.

A intervalles de temps réguliers, on prélève 0,5ml de la solution aqueuse et 0,5ml de la phase organique

et on détermine leur concentration en rhodium (en mg/l) par absorption atomique sur un spectromètre Perkin-Elmer de type M 1100.

Les résultats obtenus sont donnés dans le tableau 1 qui suit et sur la figure 1 qui représente le pourcentage d'extraction du rhodium en fonction du temps (en heures).

Au vu de cette figure, on constate qu'au bout de 16h, plus de 98% du rhodium ont été extraits avec une cinétique d'extraction lente caractéristique du rhodium.

**TABLEAU 1**

| Temps (h) | Solution aqueuse Rh (mg/l) | Rh extrait (mg/l) | Extraction (%) |
|---|---|---|---|
| 0 | 193 | - | - |
| 0,5 | 170 | 23 | 12 |
| 1 | 163 | 30 | 15,5 |
| 2 | 71 | 122 | 63,2 |
| 3 | 51,5 | 141,5 | 73,3 |
| 4 | 32,5 | 160,5 | 83,6 |
| 6 | 30,5 | 162,5 | 84,2 |
| 16 | 3,5 | 189,5 | 98,2 |

**Exemple 8** : Extraction du rhodium.

Dans cet exemple, on utilise également le composé n° 4 de l'exemple 4 pour extraire le rhodium à partir d'une solution aqueuse identique à celle de l'exemple 7, en opérant comme dans l'exemple 7, mais en utilisant comme phase organique le composé n° 4 dans du chloroforme à une concentration de 0,0539mol/l (4% en masse).

Les résultats obtenus sont donnés dans le tableau 2 qui suit et sur la figure 2 qui représente le pourcentage d'extraction du rhodium en fonction du temps.

Au vu de cette figure, on remarque qu'au bout de 16h, 74% du rhodium ont été extraits avec également une cinétique d'extraction lente caractéristique du rhodium.

**TABLEAU 2**

| Temps (h) | Solution aqueuse $Rh^{3+}$ (mg/l) | Rh extrait (mg/l) | Extraction (%) |
|---|---|---|---|
| 0 | 209 | - | - |
| 0,5 | 202 | 7 | 3,3 |
| 1 | 198 | 11 | 5,3 |
| 2 | 192,5 | 16,5 | 7,9 |
| 3 | 118 | 91 | 43,5 |
| 7 | 78,5 | 130,5 | 62,4 |
| 10 | 70,5 | 138,5 | 66,3 |
| 16 | 54,4 | 154,6 | 74 |

**Exemples 9 à 27** : Extraction du rhodium.

Dans ces exemples, on suit le même mode opératoire que dans les exemples 7 et 8 pour extraire le rhodium à partir de la même solution aqueuse en utilisant comme extractant les composés n° 1 à 6 à une concentration de 0,0539mol/l dans divers solvants organiques.

L'extractant et le solvant organique utilisés ainsi que les résultats obtenus, soit après 16h, soit après 10h sont donnés dans le tableau 3 annexé.

TABLEAU 3

| Ex. | Extractant | Diluant | Durée | % extraction du Rh |
|---|---|---|---|---|
| 9 | composé n° 1 | chloroforme | 16 h | 19 |
| 10 | " | benzonitrile | | 95 |
| 11 | " | THP/TBP | " | 15 |
| 12 | " | heptane | | 0 |
| 13 | " | hexanol | " | 43 |
| 14 | composé n° 2 | chloroforme | " | 7 |
| 15 | composé n° 2 | benzonitrile | " | 82 |
| 16 | " | hexanenitrile | " | 59 |
| 17 | " | octane-1,8-di-carbonitrile | " | 46 |
| 18 | composé n° 3 | chloroforme | " | 41 |
| 19 | composé n° 4 | chloroforme | " | 74 |
| 20 | " | benzonitrile | " | 98,2 |
| 21 | " | hexanenitrile | 10h | 74 |
| 22 | " | THP/TBP | 16h | 61 |
| 23 | " | heptane | 16h | 44 |
| 24 | " | hexanol | 16h | 73 |
| 25 | composé n° 5 | chloroforme | 16h | 53 |
| 26 | " | benzonitrile | 16h | 93 |
| 27 | composé n° 6 | chloroforme | 16h | 11 |

Au vu de ce tableau, on remarque que les meilleurs résultats sont obtenus dans le benzonitrile avec les composés 1, 2, 4 et 5 et que de bons résultats sont également obtenus avec le composé n° 4 dans le chloroforme et divers autres solvants.

**Exemple 28** : Traitement d'un concentrat de produits de fission.

Dans cet exemple, on utilise une phase organique constituée par du benzonitrile contenant le composé n° 1 de l'exemple 1 à une concentration de 0,1mol/l, pour extraire les métaux présents dans un concentrat de produits de fission provenant d'un atelier de vitrification qui a été soumis à une filtration sur filtre (0,45µm)

Le concentrat a une teneur en acide nitrique de 0,77mol/l et une teneur en nitrate de 6,3mol/l.

Pour réaliser l'extraction, on met en contact 10ml du concentrat avec 10ml de la phase organique, sous agitation au moyen d'un agitateur secoueur, pendant 16h.

On sépare alors les deux phases et on analyse la phase aqueuse pour déterminer ses teneurs en actinides (Pu, Am, Cm) par spectrométrie alpha, en radioéléments (Ru, Cs, Eu, Ce + Pr, Co) par spectrométrie gamma

et en autres éléments par spectrométrie d'émission atomique avec une source de plasma d'argon à couplage inductif (ICP).

Les résultats obtenus et la composition du concentrat sont donnés dans le tableau 4 qui suit.

**TABLEAU 4**

| | Concentrat de produits de fission | | |
|---|---|---|---|
| Elément | avant extraction | après extraction | %non extrait |
| Ce + Pr 144 | 44Ci/l (440mCi) | 42Ci/l (420mCi) | 95 |
| | 162,8.$10^{10}$Bq/l (1,62.$10^{10}$Bq) | 155,4.$10^{10}$Bq/l (1,55.$10^{10}$Bq) | |
| Ru + Rh 106 | 36Ci/l (360mCi) | 1Ci/l (10mCi) | 3 |
| | 133,2.$10^{10}$Bq/l (1,33.$10^{10}$Bq) | 3,7.$10^{10}$Bq/l (37.$10^{7}$Bq) | |
| Cs 137 | 39Ci/l (390mCi) | 38Ci/l (380mCi) | 97 |
| | 144,3.$10^{10}$Bq/l (1,44.$10^{10}$Bq) | 140,6.$10^{10}$Bq/l (1,40.$10^{10}$Bq) | |
| Cs 134 | 4 Ci/l (40mCi) | 4Ci/l (40mCi) | 100 |
| | 14,8.$10^{10}$Bq/l (0,14.$10^{10}$Bq) | 14,8.$10^{10}$Bq/l (0,14.$10^{10}$Bq) | |
| Co 60 | 0,04Ci/l (0,4mCi) | 0,04Ci/l (0,4mCi) | 100 |
| | 0,14.$10^{10}$Bq/l (14,8.$10^{6}$Bq) | 0,14.$10^{10}$Bq/l (14,8.$10^{6}$Bq) | |
| Eu 154 | 0,74Ci/l (7,4mCi) | 0,7Ci/l (7mCi) | 95 |
| | 2,73.$10^{10}$Bq/l (2,73.$10^{6}$Bq) | 2,59.$10^{10}$Bq (0,02.$10^{10}$Bq) | |
| Ru | 1,87g/l (18,7mg) | 0,09g/l (0,9mg) | 5 |
| Gd | 2,1g/l (21mg) | 2,0g/l (20mg) | 95 |
| U | 2,61g/l (26,1mg) | 0,17g/l (1,7mg) | 7 |
| Fe | 2,83g/l (28,3mg) | 2,67g/l (26,7mg) | 94 |
| Pd | 0,09g/l (0,9mg) | 0,014g/l (0,14mg) | 16 |
| Rh | 0,14g/l (1,4mg) | 0,003g/l (0,03mg) | 2 |
| Na | 7,12g/l (71,2mg) | 5,67g/l (56,7mg) | 80 |
| Pu 239 à 242 | 1,8mg/l (0,018mg) | 0,14mg/l (0,0014mg) | 8 |
| Pu 238 | 9,2mg/l (0,092mg) | 0,49mg (0,0049mg) | 5 |
| Am 241 | 0,84mg/l (8,4mg) | 0,536g/l (5,36g) | 64 |
| Cm 242 | 0,34g/l (3,4mg) | 0,17g/l (1,7mg) | 50 |
| Cm 244 | 0,125g/l (1,25mg) | 0,089g/l (0,89mg) | 71 |

Au vu de ce tableau, on remarque que l'hémisépulcrand de l'invention extrait très bien le rhodium, qu'il extrait également le ruthénium et le palladium ainsi que l'uranium et le plutonium.

**Exemple 29** : Traitement d'un concentrat de produits de fission.

Dans cet exemple on suit le même mode opératoire que dans l'exemple 28, mais on utilise comme phase organique le composé n° 4 à une concentration de 0,1mol/l (65,6g/l) dans le benzonitrile.

Le concentrat de produits de fission a une teneur en acide nitrique de 0,77mol/l et une concentration en

ions nitrates de 6,3mol/l.

La composition initiale du concentrat ainsi que les résultats obtenus après traitement de celui-ci par la phase organique sont donnés dans le tableau 5 qui suit.

**TABLEAU 5**

| | Teneur dans le concentrat de produits de fission | | |
|---|---|---|---|
| Elément | avant extraction | après extraction | % non extrait |
| Ce + Pr 144 | 44Ci/l (440mCi) | 28,2Ci/l (282mCi) | 64 |
| | $162,8.10^{10}$Bq/l ($1,62.10^{10}$Bq) | $104,34.10^{10}$Bq/l ($1,04.10^{10}$Bq) | |
| Ru + Rh 106 | 36Ci/l (360mCi) | <0,5Ci/l | 0 |
| | $133,2.10^{10}$Bq/l ($1,33.10^{10}$Bq) | <$1,85.10^{10}$Bq/l | |
| Cs 137 | 39Ci/l (390mCi) | 40Ci/l (400mCi) | 100 |
| | $144,3.10^{10}$Bq/l ($1,44.10^{10}$Bq) | $148.10^{10}$Bq/l ($1,48.10^{10}$Bq) | |
| Cs 134 | 4Ci/l (40mCi) | 4,1Ci/l (41mCi) | 100 |
| | $14,8.10^{10}$Bq/l ($0,14.10^{10}$Bq) | $15,17.10^{10}$Bq/l ($0,15.10^{10}$Bq) | |
| Co 60 | 0,04Ci/l (0,4mCi) | 0,036Ci/l (0,36mCi) | 90 |
| | $0,14.10^{10}$Bq/l ($14,8.10^{6}$Bq) | $0,13.10^{10}$Bq/l ($1,33.10^{7}$Bq) | |
| Eu 154 | 0,74Ci/l (7,4mCi) | 0,54Ci/l (5,4mCi) | 73 |
| | $2,73.10^{10}$Bq/l ($2,73.10^{6}$Bq) | $1,99.10^{10}$Bq/l ($19,98.10^{7}$Bq) | |
| Ru | 1,87g/l (18,7mg) | 0,03g/l (0,3mg) | 2 |
| Gd | 2,1g/l (21mg) | 1,31g/l (13,1mg) | 62 |
| U | 2,61g/l (26,1mg) | 0,08g/l (0,8mg) | 3 |
| Fe | 2,83g/l (28,3mg) | 2,4g/l (24mg) | 85 |
| Pd | 0,09g/l (0,9mg) | 0,003g/l (0,03mg) | 3 |
| Rh | 0,14g/l (1,4mg) | <0,5mg/l | 0 |
| Na | 7,12g/l (71,2mg) | 5,9g/l (59mg) | 82,9 |
| Pu 239 à 242 | 1,8mg/l (0,018mg) | 0,34mg/l (0,0034mg) | 19 |
| Pu 238 | 9,2mg/l (0,092mg) | 0,98mg/l (0,0098mg) | 11 |
| Am 241 | 0,84g/l (8,4mg) | 0,56g/l (5,6mg) | 67 |
| Cm 242 | 0,34g/l (3,4mg) | 0,27g/l (2,7mg) | 79 |
| Cm 244 | 0,125g/l (1,25mg) | 0,09g/l (0,9mg) | 72 |

Comme dans l'exemple 28, on remarque que l' hémisépulcrand de l'invention est un extractant efficace vis-à-vis du rhodium, du ruthénium, du palladium, de l'uranium et du plutonium.

On remarque par ailleurs que le composé n° 4, comme le composé n° 1, extraient en partie le cérium, l'europium, l'américium, le curium et le sodium. En revanche, le césium et le cobalt ne sont pas extraits.

Dans le tableau 6 qui suit, on a donné les pourcentages d'extraction des différents éléments par le composé n° 1 (cas de l'exemple 28) et par le composé n° 4 (exemple 29).

**TABLEAU 6**

| | Pourcentages extraits | |
|---|---|---|
| Elément | Ex. 28 (composé n° 1) | Ex. 29 (composé n° 4) |
| Pu 239 à 242 | 92 | 81 |
| Pu 238 | 95 | 89 |
| Am 241 | 36 | 33 |
| Cm 242 | 50 | 21 |
| Cm 244 | 29 | 28 |
| Ce + Pr 144 | 5,9 | 36 |
| Ru + Rh 106 | 97 | 100 |
| Cs 137 | 3 | 0 |
| Co 60 | 0 | 10 |
| Eu 154 | 5 | 27 |
| Ru | 95 | 98 |
| U | 93 | 97 |
| Fe | 6 | 15 |
| Pd | 84 | 97 |
| Rh | 98 | 100 |
| Na | 20 | 17 |

Au vu de ce tableau, on remarque que l'hémisépulcrand correspondant au composé n° 4 est le plus efficace.

Par ailleurs, on a constaté que la calcination de la phase organique séparée du concentrat, suivie d'une remise en solution du résidu obtenu dans de l'acide nitrique concentré, ne permet pas de récupérer la totalité des éléments extraits, sans doute en raison de la formation d'insolubles et d'amalgames avec le verre du réacteur et de la volatilisation de certains éléments.

A titre comparatif, lorsqu'on traite le même concentrat de produits de fission avec de la polyvinylpyridine, pendant 5min, à raison de 2mol/l de polyvinylpyridine, suivi d'un lavage de la polyvinylpyridine par $HNO_3$ 1M, on obtient les pourcentages d'extraction suivants dans la solution nitrique :

Rh : 92 %
Ce : 9,1 %
Cs : 9,3 %
Ru : 42 %

On remarque ainsi que l'efficacité d'extraction du rhodium est meilleure avec les hémisépulcrands de l'invention. La sélectivité pour le rhodium peut être évaluée par le rapport Rh/métal.

Par rapport au cérium et au ruthénium, on obtient une meilleure sélectivité d'extraction du rhodium avec la PV 402. En revanche, par rapport au césium, on obtient une meilleure sélectivité avec les hémisépulcrands de l'invention.

**Exemple 30** : Traitement d'un concentrat de produits de fission.

Dans cet exemple, on utilise l'hémisépulcrand de l'exemple 4 (composé n° 4) à une concentration de 0,03mol/l dans le chloroforme pour traiter un concentrat de produits de fission.

Le mode opératoire utilisé est représenté schématiquement sur la figure 3.

Sur cette figure, on voit que les opérations consistent à effectuer une extraction suivie par 2 lavages acides de la phase organique chargée.

Dans ce but, au stade d'extraction (1), on met en contact, pendant une durée variable dans un agitateur

secoueur, 10ml de la phase organique d'extraction et 10ml de concentrat de produits de fission, puis on sépare les deux phases et on analyse la phase aqueuse en (3) pour déterminer sa composition en utilisant les mêmes méthodes d'analyse que dans l'exemple 28.

On effectue ensuite (en 5) un premier lavage de la phase organique (7ml) par 10ml de $HNO_3$ à 1mol/l, puis (en 7) un second lavage de la phase organique (6ml) par 10ml de $HNO_3$ à 1mol/l et on analyse (en 3) les phases aqueuses issues des lavages pour déterminer leurs teneurs en actinides par spectrométrie alpha, leurs teneurs en radioéléments par spectrométrie gamma et leurs teneurs en éléments pondérables par émission atomique ICP.

Les résultats obtenus après 30min, 60min, 180min et 300min de mise en contact au stade d'extraction, sont donnés dans le tableau 7 et sur la figure 4.

TABLEAU 7

| Elément | Concentrat de produits de fission | Durée d'extraction | Solvant organique après extraction | | Phase aqueuse après 1er lavage | | Phase aqueuse après 2ème lavage | |
|---|---|---|---|---|---|---|---|---|
| | | | teneur | % extrait | teneur | % extrait | teneur | % extrait |
| Rh | 0,19g/l | 30min | 0 | 0 | 0 | | 0 | |
| | | 60min | 0 | 0 | 0 | | 0 | |
| | | 180min | 0,03g/l | 15,8 | | | | |
| | | 300min | 0,11g/l | 57,9 | 0,0017g/l | 1,3 | | |
| Pd | 0,01g/l | 30min | > | > 50 | | | | |
| | | 60min | 0,005g/l* | > 50 | | | | |
| | | 180min | > 0,005g/l | > 50 | | | | |
| | | 300min | > 0,005g/l - | - | < 0,001g/ | | | |
| U | 0,87g/l | 30min | 0,07g/l | 8 | | | | |
| | | 60min | 0,45g/l | 51,7 | | | | |
| | | 180min | 0,83g/l | 95,4 | | | | |
| | | 300min | 0,869g/l | 99,9 | 0,35g/ll | 57,5 | | |
| Pu 239-242 | 5,8mg/l | 60min | 1,28mg/l | 22,1 | 0,53mg/l | 13,1 | 0,25mg/l | 7,2 |
| | | 180min | 3,3mg/l | 56,9 | 0,98mg/l | 24,1 | 0,41mg/l | 11,8 |
| | | 300min | 2,24mg/l | 38,6 | 2,73mg/l | 67,2 | 0,29mg/l | 8,3 |
| Ru 106 | 19Ci/l | 60min | 2,42Ci/l | 12,7 | 260mCi/l | 2 | 21mCi/l | 0,18 |
| | | 180min | 8,25Ci/l | 43,4 | 1,37Ci/l | 10,3 | 570mCi/l | 5% |
| | | 300min | 15,7Ci/l | 82,6 | 3,4Ci/l | 25,6 | 0,094Ci/l | 0,82 |

* limite de détection du palladium dans ce milieuu organique

Sur la figure 4, on a illustré les pourcentages d'extraction de Rh, Pd, U, Pu, et Ru en fonction du temps

de contact.

Au vu de cette figure, on remarque que la cinétique d'extraction du rhodium est la plus lente puisqu'il faut 5h de contact pour extraire 58% du rhodium. Le palladium est très vite extrait puisqu'après 30min de contact, il n'est plus détecté par émission atomique ICP dans le concentrat. Après 300min de contact, le palladium semble se décrocher au profit de l'uranium, du rhodium, du ruthénium. Pour l'uranium, le rhodium, le ruthénium et le plutonium, les quantités extraites augmentent avec le temps de contact. Ainsi, en jouant sur le temps de contact, on peut séparer l'uranium du rhodium. En effet, après deux extractions d'une heure, l'uranium peut être extrait en totalité, sans coextraction du rhodium. Par ailleurs, on remarque que le lavage de la phase organique à l'acide nitrique est favorable à la purification du rhodium car il permet de réextraire les radioéléments, le plutonium et l'uranium sans perte importante du rhodium.

Si l'on effectue le même traitement mais en utilisant comme phase organique une solution de trithia-6,9,12-heptadécane dans le chloroforme, on obtient une extraction du palladium et non du rhodium.

Ainsi, l'utilisation des hémisépulcrands de l'invention est très intéressante car elle permet une très bonne extraction du rhodium à partir des solutions issues du retraitement des combustibles nucléaires irradiés telles que les concentrats de produits de fission.

## Revendications

1. Hémisépulcrand soufré de formule :

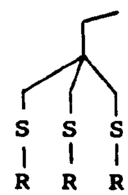

(I)

dans laquelle R représente un groupe choisi parmi
- $(CH_2)_pH$
- $(CH_2)_m$-S-$(CH_2)_pH$
- $(CH_2)_m$-S-$(CH_2)_n$-S-$(CH_2)_pH$

avec m et n qui peuvent être identiques ou différents représentant un nombre entier égal à 2,3,4 ou 5 et p étant un nombre entier allant de 1 à 18.

2. Hémisépulcrand selon la revendication 1, caractérisé en ce que R représente -$(CH_2)_pH$ avec p=5 ou 8.

3. Hémisépulcrand selon la revendication 1, caractérisé en ce que R représente -$(CH_2)_m$-S-$(CH_2)_pH$ avec m=2,3 ou 4 et p=8.

4. Hémisépulcrand selon la revendication 1, caractérisé en ce que R représente -$(CH_2)_m$-S-$(CH_2)_n$-S-$(CH_2)_pH$ avec m=n=2 et p=1.

5. Procédé de préparation d'un hémisépulcrand soufré de formule :

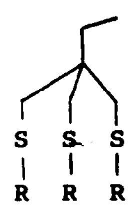

(I)

dans laquelle R représente -$(CH_2)_pH$ avec p étant un nombre entier allant de 1 à 18, caractérisé en ce que l'on fait réagir le tritosylate de 1,1,1-tris(hydroxyméthyl)propane avec un thiol de formule $H(CH_2)_p$-SH dans laquelle p a la signification donnée ci-dessus.

**6.** Procédé de préparation d'un hémisépulcrand soufré de formule :

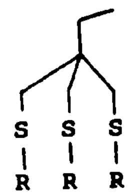

(I)

dans laquelle R représente
$-(CH_2)_m-S-(CH_2)_pH$
avec m=2,3,4 ou 5 et p étant un nombre entier de 1 à 18, caractérisé en ce que l'on fait réagir un dithiol de formule $HS-(CH_2)_m-SH$ avec un halogénure de formule $X(CH_2)_pH$ dans lesquelles m et p ont les significations données ci-dessus et X représente I ou Br, puis on fait réagir le produit obtenu avec le tritosylate de 1,1,1-tris(hydroxyméthyl)propane.

**7.** Procédé de préparation d'un hémisépulcrand soufré de formule :

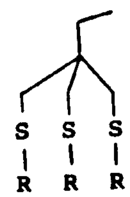

(I)

dans laquelle R représente $-(CH_2)_m-S-(CH_2)_n-S-(CH_2)_pH$ avec m et n, identiques ou différents, étant égaux à 2,3,4 ou 5 et p étant un nombre entier allant de 1 à 18,
caractérisé en ce que l'on fait réagir un dithiol de formule
$HS-(CH_2)_m-S-(CH_2)_n-SH$
avec un halogénure de formule $X(CH_2)_pSH$ dans lesquelles m, n et p ont les significations données ci-dessus et X représente I ou Br, puis on fait réagir le produit obtenu avec le tritosylate de 1,1,1-tris(hydroxyméthyl)propane.

**8.** Procédé de séparation d'au moins un métal choisi parmi Rh, Pd, Ru, U et Pu, présents dans une solution aqueuse issue du retraitement de combustibles nucléaires irradiés, caractérisé en ce que l'on met en contact la solution aqueuse avec une phase organique immiscible comprenant un hémisépulcrand soufré selon l'une quelconque des revendications 1 à 4, pour extraire le ou les métaux dans la phase organique.

**9.** Procédé selon la revendication 8, caractérisé en ce que la phase organique immiscible est une phase liquide comprenant un diluant organique.

**10.** Procédé selon la revendication 9, caractérisé en ce que le diluant organique est choisi dans le groupe comprenant le chloroforme, le benzonitrile, l'hexane nitrile et l'hexanol.

**11.** Procédé selon l'une quelconque des revendications 9 et 10, caractérisé en ce que la concentration en hémisépulcrand soufré de la phase organique est de 0,025 à 0,5mol/l.

**12.** Procédé selon l'une quelconque des revendications 8 à 10, caractérisé en ce que la solution aqueuse est une solution nitrique.

**13.** Procédé selon l'une quelconque des revendications 8 à 12, caractérisé en ce que l'hémisépulcrand soufré est le 3,3'-bis(2,7-dithiapentadécane)-5,10 dithiaoctadécane.

**14.** Procédé selon l'une quelconque des revendications 8 à 13, caractérisé en ce que la solution aqueuse est un concentrat de produits de fission.

**15.** Procédé selon l'une quelconque des revendications 8 à 14, caractérisé en ce que le métal est le rhodium.

**16.** Procédé selon la revendication 15, caractérisé en ce que l'on soumet de plus la phase organique ayant extrait le rhodium à au moins un lavage par une solution aqueuse nitrique ayant une concentration en $HNO_3$ de 0,1 à Imol/l.

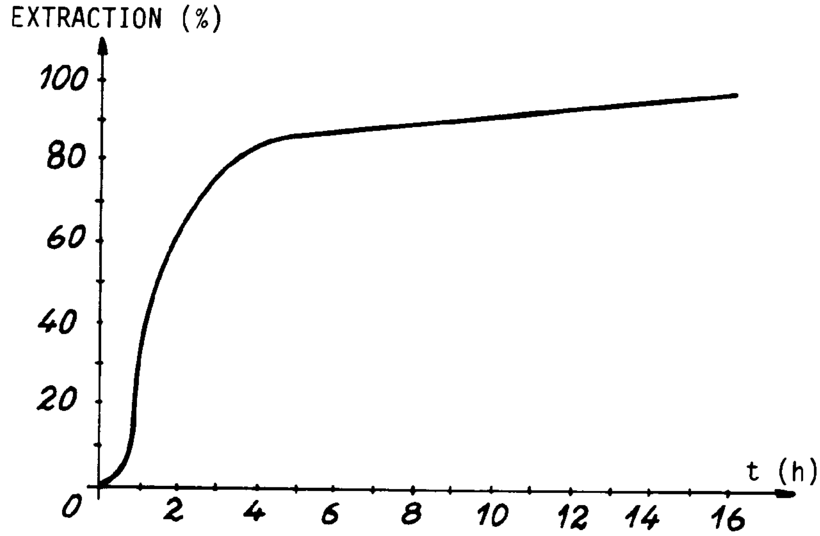


FIG. 1

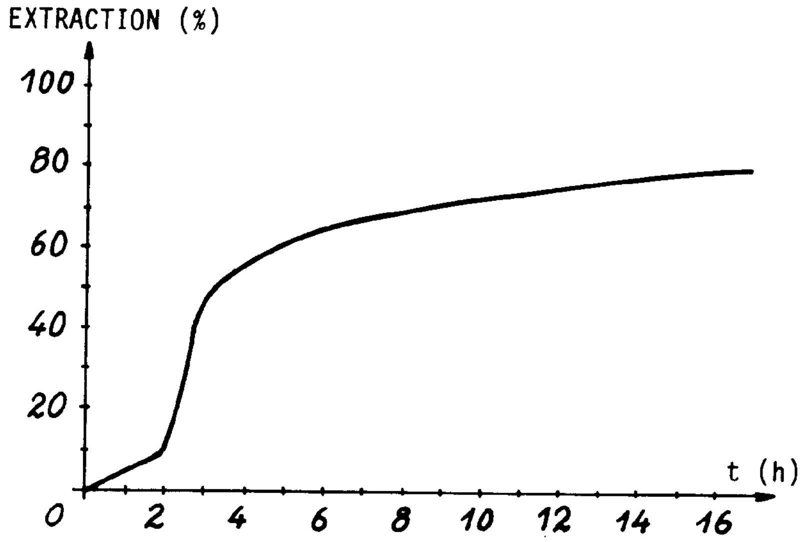


FIG. 2

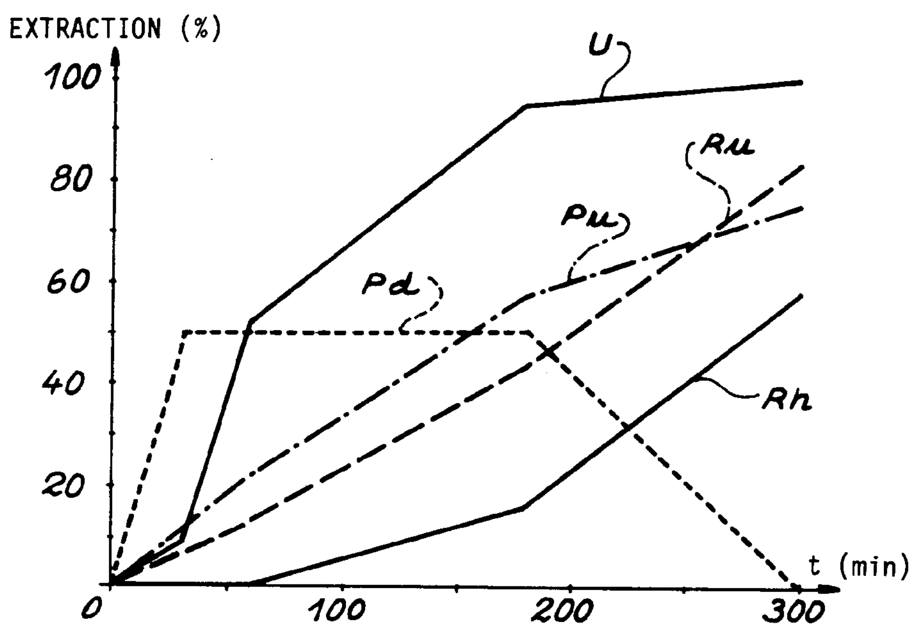


FIG. 4

FIG. 3

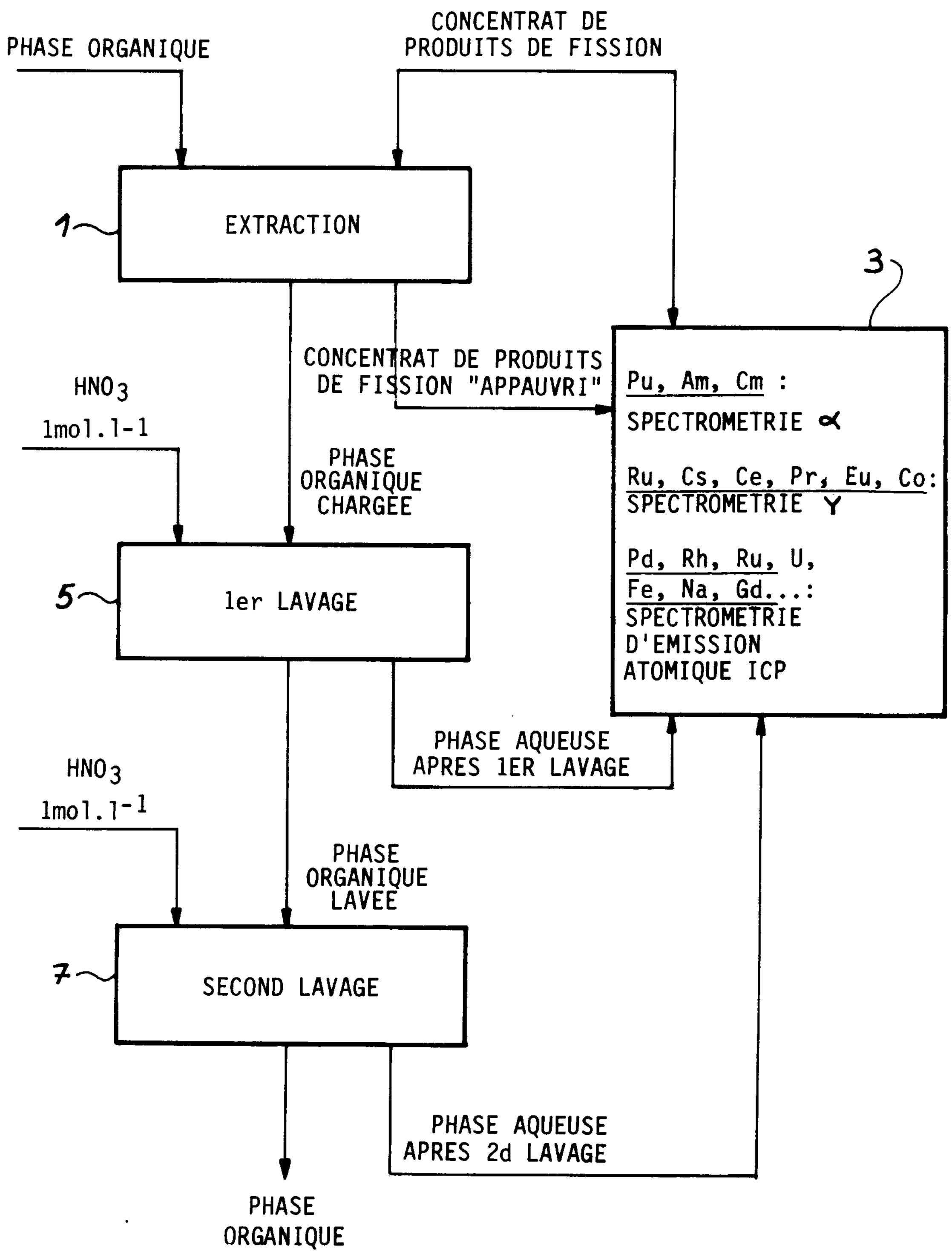
PHASE ORGANIQUE
CONCENTRAT DE PRODUITS DE FISSION
1
EXTRACTION
3
CONCENTRAT DE PRODUITS DE FISSION "APPAUVRI"
Pu, Am, Cm :
SPECTROMETRIE α
$HNO_3$
1mol.l-1
PHASE ORGANIQUE CHARGEE
Ru, Cs, Ce, Pr, Eu, Co:
SPECTROMETRIE γ
Pd, Rh, Ru, U,
Fe, Na, Gd...:
SPECTROMETRIE D'EMISSION ATOMIQUE ICP
5
1er LAVAGE
PHASE AQUEUSE APRES 1ER LAVAGE
$HNO_3$
$1mol.l^{-1}$
PHASE ORGANIQUE LAVEE
7
SECOND LAVAGE
PHASE AQUEUSE APRES 2d LAVAGE
PHASE ORGANIQUE

Office européen des brevets

# RAPPORT DE RECHERCHE EUROPEENNE

Numero de la demande
EP 94 40 2611

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | GB-A-2 058 063 (AGFA-GEVAERT AG)<br>* revendications 18,21,44 * | 1 | C07C321/14<br>C22B3/00<br>C22B60/02 |
| A<br><br><br>D | EP-A-0 459 854 (COGEMA (COMPAGNIE GENERALE DES MATIERES NUCLEAIRES))<br>* le document en entier *<br>& FR-A-2 662 159 | 1,8-12, 14-16 | |
| A<br><br><br>D | EP-A-0 559 536 (COMPAGNIE GENERALE DES MATIERES NUCLEAIRES (COGEMA))<br>* le document en entier *<br>& FR-A-2 688 335 | 8 | |
| A | SOVIET ATOMIC ENERGY,<br>vol.64, no.2, 1988<br>pages 119 - 126<br>V. S. SHMIDT ET AL. 'Palladium in spent power station fuel solvent-extraction reprocessing'<br>* le document en entier * | 8 | |
| | | | DOMAINES TECHNIQUES RECHERCHES (Int.Cl.6) |
| A | CHEMISCHE BERICHTE,<br>vol.111, no.1, 1978, WEINHEIM DE<br>pages 132 - 142<br>KLAUS BRODERSEN ET AL. 'Über neue mehrzähnige Schwefel-Liganden...'<br>* page 140 * | 1 | C07C<br>C22B |
| A | JOURNAL OF THE CHEMICAL SOCIETY, CHEMICAL COMMUNICATIONS,<br>no.4, 1987, LETCHWORTH GB<br>pages 306 - 307<br>CELIA M. THORNE ET AL. 'A new approach to hexakis(thioether) Co-ordination'<br>* page 307 * | 1 | |

Le présent rapport a été établi pour toutes les revendications

| Lieu de la recherche | Date d'achèvement de la recherche | Examinateur |
|---|---|---|
| BERLIN | 4 Janvier 1995 | Rufet, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)